# EUROPEAN PATENT APPLICATION

(11) **EP 3 932 415 A1**
(43) Date of publication of application: **05.01.2022**
(21) Application number: 20382592.2
(22) Date of filing: 01.07.2020
(51) Int. Cl.: A61K 35/74, A61K 35/745, A61K 35/747, A61P 25/00, A61P 25/28

(54) **GUT MICROBIOTA COMPOSITION AND USES THEREOF**

(71) Applicant: Fundació Institut d'Investigació Biomèdica de Girona Dr. Josep Trueta (IDIBGI), 17190 Salt (Girona) (ES); Universitat Pompeu Fabra, 08002 Barcelona (ES); Consorcio Centro de Investigación Biomédica en Red, M.P., 28029 Madrid (ES); Universitat de València, 46010 Valencia (ES); Fundación para el Fomento de la Investigación Sanitaria y Biomédica de la Comunitat Valenciana (FISABIO), 46020 Valencia (ES)
(72) Inventor: Maldonado López, Rafael, 08002 Barcelona (ES); Burokas, Aureliju, 29029 Madrid (ES); Moya Simarro, Andrés, 46010 Valencia (ES); Pérez Brocal, Vicente, 46020 Valencia (ES); Fernández-Real Lemos, José Manuel, 17003 Girona (ES); Mayneris Perxachs, Jordi, 28029 Madrid (ES); Arnoriaga Rodríguez, Maria, 17002 Girona (ES)
(74) Representative: Clarke Modet & Co.

(57) **Abstract**

The present invention is a gut microbiota composition comprising at least one species of genus *Clostridium* or *Tissierella* (family Clostridiaceae), *Lactobacillus* (family Lactobacillaceae), *Ruminococcus* (family Ruminococcaceae); *Megamonas* and *Anaerovibrio* (family Selenomonadaceae), *Acetitomaculum* and *Roseburia* (family Lachnospiraceae), *Megasphaera* (family Veillonellaceae), *Leuconostoc* (family Leuconostocaceae), *Sutterella* or the species *Dakarella massiliensis* (family Sutterellaceae), *Schwartzia succinivorans* (family Selenomonadaceae) or *Firmicutes bacterium CAG:95.* for the specific prevention and/or treatment of a mental disorder with memory impairment in a subject, and the form of administration of said microbiota composition.

## Description

### Field of the invention

The present invention refers to the field of nutraceuticals and medicine. In particular, to the field of neurology and to the capability of ameliorating and/or treating cognitive diseases and disorders, and to the improvement of cognitive abilities.

### Background of the invention

There is an emerging and consistent link between the gut microbiota (the billions of bacteria living in the intestine) and different health conditions, weight gain, exercise, sleep, skin appearance and many other correlations being investigated, including the mental health.

Related to this, recent evidence in mice shows a potential link between the gut microbiota and the brain. The gut microbiota is increasingly recognized to be associated with cognitive traits and memory. Further, the same agents that have shown to delay or prevent cognitive impairment (weight loss, antidiabetic drugs) are well known to cause microbiota shifts.

The decline of neurocognitive function is currently among the greatest health concerns of old age. There is preliminary evidence that some bacteria species live within the human brain.

Although the study of the gut microbiota brain axis evidence is scarce in humans, several compositions comprising microorganisms for the treatment of diseases have been proposed.

The application WO 2014/070016 A2 discloses a nutritional supplement or composition comprising *Bifidobacterium breve* and non-digestible oligosaccharides to prevent and/or treat a decrease cognitive performance and neuroinflammation in infants, from results obtained in the behaviour of mice.

Also, WO 2016/065419 A1 describes the use of a composition comprising orotic acid and probiotic microorganisms for the treatment of depression, anxiety or a depressive or anxiety-related disorder. The microorganisms of preference are *Lactobacillus, Bifidobacterium, Lactococcus* and *Streptococcus.*

WO 2018/234994 A1 also uses *Lactobacillus, Bifidobacterium, Lactococcus* and *Streptococcus* in a composition for the improvement of several pathologies, including biological aging processes that lead to a progressive loss of memory and the ability to concentrate. The document analyses the activity of the microorganisms on PBMCs isolated from the blood of patients affected by Parkinson's disease in order to assess the effects of the microorganisms on oxidative stress. A strain of *Lactobacillus salivarius* and another of *Lactobacillus plantarum* were revealed as preferred.

The application CA 3063669 A1 is considered to be the closest prior art to the present invention. The document teaches about a method of diagnosis of a neurological mental disorder comprising measuring the level of microorganisms comprising proteobacteria, bifidobacteria and/or firmicutes, among others, in the intestine; in particular of *Klebsiella oxytoca, Escherichia coli* and *Morganella morganii* as proteobacteria, and *Bifidobacterium animalis.* A decrease in the level of these microorganisms would mean the presence of the disease. No indication is given, however, to the use of these species in the treatment of the mental disease - which is consistent, as *Klebsiella oxytoca* and *Escherichia coli* can render pathogenic.

It is well known that a deficit in a particular species of a bacterial ecosystem cannot be understood as if the reposition of the said species would be for the direct benefit of the organism. This is rather a concern about the relative proportion of the different species in the intestine of the healthy subject. Besides, the microbiota associated to a disease is probably different in every region of the world, related to the different diet regimes. This is why CA 3063669 A1 does not indicate or could even suggest that the species they identified for the diagnosis could be of any benefit in the treatment of the mental disorder. Indeed, the document proposes other species for a composition useful in the prevention and/or treatment of the neurological mental disorder, in particular *Lactobacillus reuteri* and *Bifidobacterium adolescentis.*

Fungi are also present in the gut microbiota. Some fungi have been reported for the treatment of related intestine diseases, for example at the application CN 105434524 A. In this sense, CN 104524188 A further teaches about the use of specific fungi for improving the cognitive function of vascular dementia.

There is also evidence of at least an intestinal fungus commercialized for the treatment of a disease; in particular, ULTRA LEVURA^{®}, which is a pharmaceutical composition containing *Saccharomyces boulardii* yeast for the treatment of diarrhea.

The problem of the art can be formulated as the obtention of a composition of gut microbiota for the specific prevention and/or treatment of a mental disorder with memory impairment.

The solution proposed by the present invention is a microbiota composition comprising at least one species of the genera *Clostridium* or *Tissierella* (family Clostridiaceae), *Lactobacillus* (family Lactobacillaceae), *Ruminococcus* (family Ruminococcaceae); *Megamonas* and *Anaerovibrio* (family Selenomonadaceae), *Acetitomaculum* and *Roseburia* (family Lachnospiraceae), *Megasphaera* (family Veillonellaceae), *Leuconostoc* (family Leuconostocaceae), *Sutterella* or the species *Dakarella massiliensis* (family Sutterellaceae), *Schwartzia succinivorans* (family Selenomonadaceae) or *Firmicutes bacterium CAG:95.*

### Description of the invention

The present invention is a gut microbiota composition comprising one or more species of genus *Clostridium* (family Clostridiaceae), *Lactobacillus* (family Lactobacillaceae), *Megamonas* and *Anaerovibrio* (family Selenomonadaceae), or *Roseburia* (family Lachnospiraceae) of phylum Firmicutes, for the prevention and/or treatment of a mental disorder with memory impairment in a subject.

In the present invention, the term "microbiota" means an ecological community of commensal, symbiotic and/or pathogenic microorganisms found in and on all multicellular organisms from plants to animals. Microbiota includes bacteria, protists, fungi and viruses.

In the present invention, the term "gut microbiota" refers to a microbiota comprising fungi and bacteria present in a digestive tract, in particular specifically present in the intestines.

In the present invention, a "mental disorder with memory impairment" is understood as a disorder with attenuations in short-term memory (the capacity to recognise, recall and retrieve information shortly after its occurrence), which is divided into subsystems for verbal and visual information.

Another aspect of the invention is a gut microbiota composition, comprising one or more species of genus *Clostridium* (family Clostridiaceae); *Ruminococcus* (family Ruminococcaceae); *Megamonas* or *Anaerovibrio* (family Selenomonadaceae); *Acetitomaculum* (family Lachnospiraceae); or the species *Schwartzia succinivorans* (family Selenomonadaceae) or *Firmicutes bacterium CAG:95* for the prevention and/or treatment of a mental disorder with memory impairment in the verbal memory in a subject.

In the present invention, "verbal memory" refers to memory for verbally presented information.

Another aspect of the invention is a gut microbiota composition, comprising one or more species of genus *Ruminococcus* (family Ruminococcaceae), *Megasphaera* (family Veillonellaceae) or *Anaerovibrio* (family Selenomonadaceae), for the prevention and/or treatment of a mental disorder with memory impairment in the immediate memory in a subject.

In the present invention, "immediate memory" refers to a type or stage of **memory** in which an individual recalls information immediately presented, although this information may be forgotten after its use.

Another aspect of the invention is a gut microbiota composition, comprising one or more species *Lactobacillus rhamnosus* or *L. helveticus,* for the prevention and/or treatment of a mental disorder with memory impairment in the non-spatial memory in a subject.

In the present invention, "non-spatial memory" refers to not spatial matter; this meaning not relating to, occupying, or having the character of space nonspatial data or not relating to or involved in the perception of relationships as of objects in space.

Another aspect of the invention is a gut microbiota composition, comprising one or more species of genus *Tissierella* (family Clostridiaceae) for the prevention and/or treatment of a mental disorder with memory impairment in the memory function in a subject.

Another aspect of the invention is a gut microbiota composition, comprising one or more species of *Dakarella massiliensis* or *Sutterella* (family Sutterellaceae), or *Leuconostoc* (family Leuconostocaceae), for the prevention and/or treatment of a mental disorder with memory impairment in the memory function in a subject.

In the present invention, "memory function" refers to the faculty of the brain by which data or information is encoded, stored, and retrieved when needed. It is the retention of information over time for the purpose of influencing future action.

In particular, the mental disorder with memory impairment may be one or more selected from a group including dementia, Alzheimer's disease, Parkinson's disease or Huntington's disease, mood disorders as anxiety or depression, insomnia, delusional disorder, obsessive disorder, migraine, stress, memory deficit, deficit in the executive capacity, cognitive disorder and disturbance attention. There are no results in the art that could be specifically applicable to these disorders.

The bacterial species disclosed for the use of the present invention have not been identified in the art for the prevention or treatment of a mental disorder with memory impairment. The composition for use should render inventive, as no suggestion has been given about the use of a microbiota composition capable for reaching a relative proportion of the different species of bacteria in the intestine of a subject that could be of benefit for the prevention and/or treatment of said mental disorder.

Another preferred aspect is the use of the gut microbiota composition of the present invention in the preparation of a medicament for the prevention and/or treatment of a mental disorder with memory impairment. Another aspect is a method of prevention and/or treatment of a mental disorder with memory impairment comprising administering a composition of the present invention to a subject in need thereof.

Another aspect of the present invention is the microbiota composition for use in the improvement of the cognitive abilities of a subject. Another aspect is the use to ameliorate cognitive disorders and/or the improvement of cognitive abilities. Another aspect is the preparation of a medicament thereto. Still another aspect is a method for prevention, amelioration and/or treatment of a cognitive disorder and/or for the improvement of cognitive abilities comprising administering a composition of the present invention to a subject in need thereof.

Preferably, said subject is a mammal, even more preferably a human.

Another aspect refers to intestinal microbiota from a healthy subject for use in the prevention, amelioration and/or treatment of cognitive disorders and/or for the improvement of cognitive abilities. Another aspect is the preparation of a medicament thereto. Another aspect is the therapeutically effective dose.

In another preferred aspect, the cognitive abilities is an improvement in the memory or executive capacity, more preferably memory, even more preferably the improvement in the memory is an improvement in immediate memory and/or in short-term memory.

In the present invention, the healthy subject does not have a cognitive disorder, but on the contrary improved cognitive status and/or cognitive abilities than the subject in need of the treatment. Preferably, the healthy subject has improved memory and/or executive capacity than the subject in need thereof, more preferably the healthy subject has improved immediate memory and/or short-term memory than the subject in need of the treatment.

In another aspect, the microbiota composition comprises at least 100,000 microorganisms per mL, more preferably, at least 200,000 microorganisms per mL, even more preferably, at least 500,000 microorganisms per mL.

The microbiota composition is presented in a form suitable for oral administration, in solid, liquid or powder; even more preferably, liquid. In one embodiment, the administration of the composition to the human subject takes place orally, for example in the form of a pill, tablet, a capsule, solution, suspension, syrup, food containing the probiotic bacteria, or in any other form known to the person skilled in the art.

In the present invention, a "pharmaceutically acceptable excipient" is a substance or a combination of substances that render auxiliary to a pharmaceutical, dietary or nutraceutical compound as active ingredient to be found in a composition, and perfectly known by the expert. Their function is to affect the stability, modify the release or having an effect in other feature of the composition. Non-limiting examples of excipients, are diluents, absorbents, lubricants, colourants, surfactants, antioxidants, sweeteners, binders, disaggregating agents and others.

The results of the present application show how the gut microbiota of the healthy subjects with increased memory scores contain increased relative abundance of bacteria belonging to Anaerolineae, Clostridiaceae and unclassified families of Rhodospirillales (phylum Proteobacteria, class Alphaproteobacteria); and fungi belonging to phylum Microsporidia. It is also also observed an increased relative abundance of bacteria belonging to unassigned Blastocatellia class (phylum Acidobacteria) and Magnetococcaceae and uncultured bifidobacteria in humans or mice with increased memory scores.

### Analysis of the Gut Metagenome discloses bacterial gene functions and species associated with memory scores

Memory function was evaluated in a cohort of 116 middle-aged subjects, 65 of them with obesity (Table 1).

### Table 1

**Table 1. Results are expressed as number and frequencies for categorical variables, mean and standard deviation (SD) for normal distributed continuous variables and median and interquartile range [IQ] for non-normal distributed continuous variables. To determine differences between study groups, χ² for categorical variables were used, unpaired Student's t-test in normal quantitative and Mann-Whitney U test for non-normal quantitative variables. P value determinates differences between subjects with obesity (Body mass index, BMI ≥ 30 kg/m²) and without obesity (BMI 18.5-30 kg/m²). SBP, systolic blood pressure; DBP, diastolic blood pressure; HDL-C, high density lipoprotein cholesterol; FPG, fasting plasma glucose; HbA1c, glycated haemoglobin; hsCRP, high-sensitive C-reactive protein; CVLT, California Verbal Learning Test; IR, Immediate Recall; SDFR, Short Delayed Free Recall; PHQ-9, Patient Health Questionnaire.**

| *Clinical and neuropsychological data of the human discovery cohort.* | | | | |
|---|---|---|---|---|
| | Total population (n=116) | Without obesity (n=51) | With obesity (n=65) | *p* |
| Age (years) | 50.4 [41.8-58.5] | 53.9 [44.4-59.0] | 48.6 [41.1-57.1] | 0.097 |
| Females n (%) | 81 (69.8) | 34 (66.7) | 47 (72.3) | 0.511 |
| Education (years) | 12 [11-16.8] | 15 [12-17] | 12 [9-14] | 9.0x10⁻¹ |
| BMI (kg/m²) | 34.8 [25.3-43.3] | 24.6 (2.6) | 43.2 (6.7) | 3.3x10⁻³⁴ |
| Waist (cm) | 110 [92-126] | 89.8 (9.6) | 125.2 (13.9) | 3.6x10⁻²⁹ |
| Fat mass (%) | 43.6 [34-50.5] | 32.4 (7.2) | 49.9 (5.5) | 2.7x10⁻²⁷ |
| SBP (mmHg) | 132.8 (20.0) | 124.3 (15.8) | 139.3 (20.6) | 2.3x10⁻⁵ |
| DBL (mmHg) | 74.8 (11.5) | 71.2 (10.9) | 77.6 (11.3) | 0.003 |
| HDL-C (mg/dL) | 56 [45-68] | 66.0 (17.0) | 50.8 (12.7) | 2.1x10⁻⁷ |
| Triglycerides (mg/dL) | 90 [65.3-134.8] | 79 [58-96] | 123 [81.5-156] | 7.1x10⁻⁵ |
| FPG (mg/dL) | 96 [90-102.8] | 95 [89-101] | 97 [92.5-104.5] | 0.196 |
| HbA1c (%) | 5.5(0.3) | 5.5 (0.3) | 5.6 (0.3) | 0.035 |
| hsCRP (mg/dL) | 2.4 [0.7-5.9] | 0.7 [0.4-1.4] | 5.0 [2.7-9.5] | 8.1x10⁻¹⁴ |
| CVLT IR (score) | 61 [55-67.8] | 65 [56-70] | 59 [52.5-65] | 0.003 |
| CVLT SDFR (score) | 14 [12-15] | 14 [12-16] | 13 [11-14] | 0.002 |
| Total Digit Span (score) | 14 [11.3-17] | 15 [13-18] | 13 [11-16] | 0.003 |
| PHQ-9 (score) | 5.5 [3-9] | 4 [2-6] | 7 [4-10] | 1.8x10⁻⁴ |

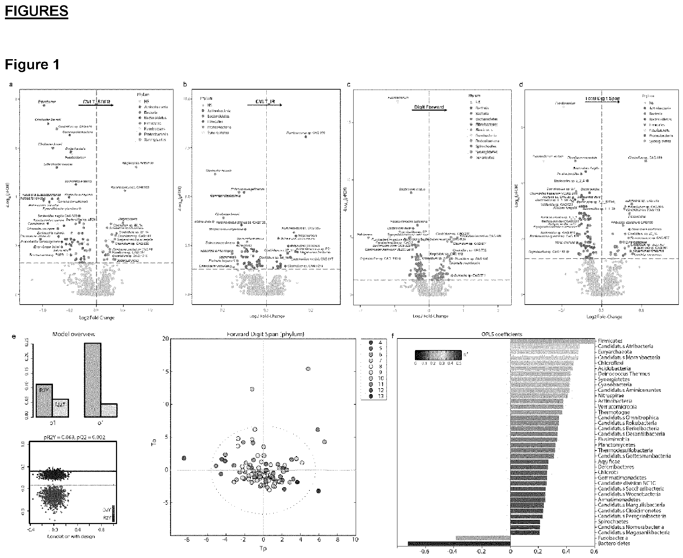

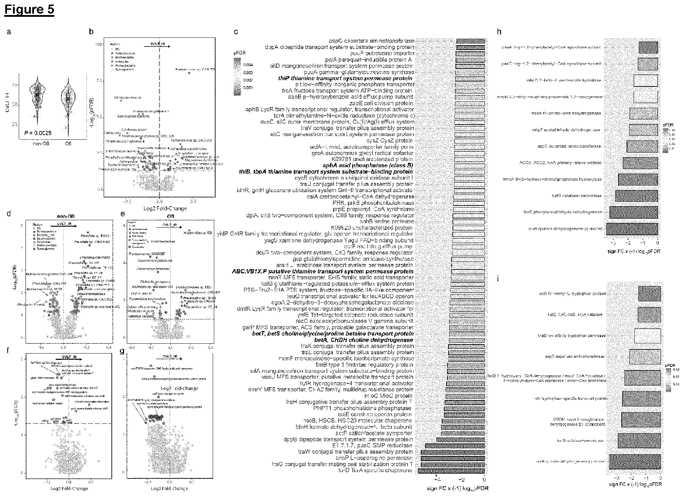

Impairments on learning and immediate recall, short delayed recall and working memory were observed among subjects with obesity, based on the scores of California Verbal Learning Test Immediate Recall (CVLT-IR), California Verbal Learning Test Short Delayed Free Recall (CVLT-SDFR) and Total Digit Span (TDS), respectively (Table 1; Figure 1a and 1b; Figure 5a).

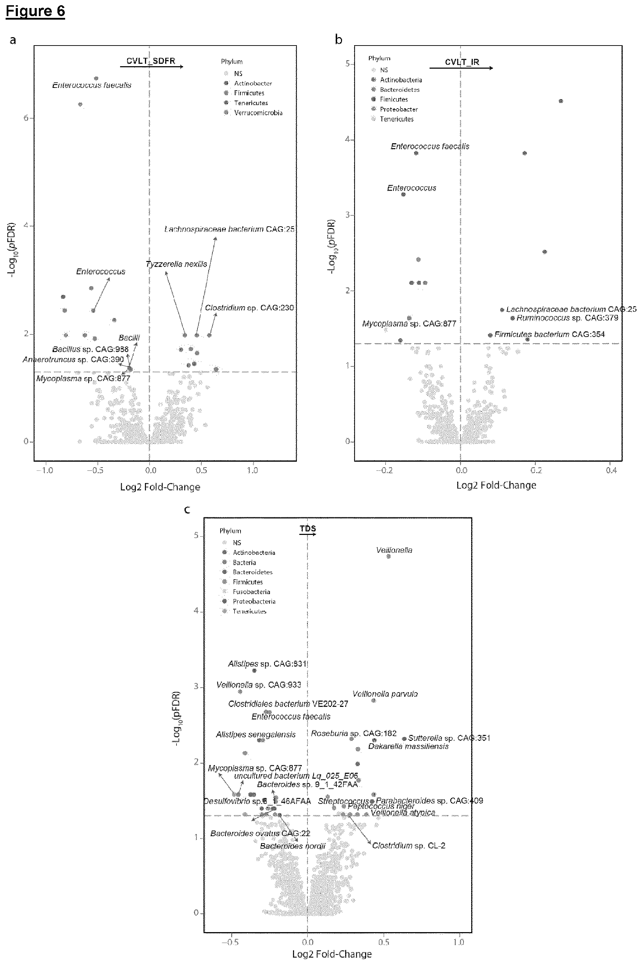

A characteristic microbiome ecosystem was associated with cognitive scores using DESeq2, after adjusting for age, sex, body mass index, years of education and depression scores assessed using the Patient Health Questionnaire (PHQ)-9 (Figure 1c-f, Figure 5b-c). Common species were positively associated with learning and verbal memory [CVLT-SDFR (Figure 1c), CVLT-IR (Figure 5b)] and working memory [TDS (Figure 1d], such as *Clostridium* sp. 27_14, *Megamonas funiformis, Clostridium* sp. CAG:230, all of them belonging to Firmicutes phylum. On the contrary, negative associations between the gut microbiota and memory scores were identified within the phylum Bacteroides (*Bacteroides fragilis* CAG:558, *Bacteroides* sp. 1_1_14, *Bacteroides* sp. 43_46, *Bacteroides caccae* CAG:21, *Bacteroides* sp. 2_1_16, *Bacteroides* sp. HMSC067B03, *Bacteroides* sp. AR20), phylum Fusobacteria *(Fusobacterium* sp. HMSC073F01, uncultured *Fusobacterium, Fusobacterium varium),* phylum Proteobacteria (*Citrobacter freundii, Enterobacter cloacae, Salmonella enterica, Klebsiella aerogenes*) and some Firmicutes (*Coprobacillus* sp. 3_3_56FAA, *Coprobacillus* sp. D7, *Erysipelatoclostridium ramosum, Ruminococcus gnavus* CAG:126).
Of note, while some species were positively and specifically associated with verbal learning such as *Ruminococcus* sp. CAG:353, *Megasphaera, Roseburia sp.CAG:197,* and *Anaerovibrio* sp. RM50 (Figure 1c, Figure 5b), others were positively linked to working memory but not with learning or verbal memory (*Clostridium* sp. CAG:440, *Ruminococcus* sp. CAG:177, *Lactobacillus lactis, L. rhamnosus*) (Figure 1d), indicative of divergent memory domains. Of note, several of the identified bacterial species, were also longitudinally associated with the several memory domains measured one year later (Figure 6). The characteristics of these subjects are shown in Table 2.

### Table 2

**Table 2. Results are expressed as median and interquartile range [IQ]. To determine differences between study groups, paired Mann-Whitney U test were used. BMI, body mass index; SBP, systolic blood pressure; DBP, diastolic blood pressure; HDL-C, high density lipoprotein cholesterol; FPG, fasting plasma glucose; HbA1c, glycated haemoglobin; hsCRP, high-sensitive C-reactive protein; CVLT, California Verbal Learning Test; IR, Immediate Recall; SDFR, Short Delayed Free Recall; PHQ-9, Patient Health Questionnaire.**

| *Clinical and neuropsychological data of the human follow-up cohort.* | | | |
|---|---|---|---|
| Total population (Female n=47, 68.1%) | Baseline (n=69) | Follow-up (n=69) | *p* |
| Age (years) | 51.9 [44.3-59] | 53 [45.4-60.2] | 5.2x10⁻¹³ |
| BMI (kg/m²) | 28.2 [24.7-40.0] | 28 [24.9-36.4] | 0.192 |
| Waist (cm) | 103 [86.3-121.3] | 97 [87-119] | 0.044 |
| Fat mass (%) | 40.2 [32.7-49.7] | 36.9 [31.8-46.9] | 0.158 |
| SBP (mmHg) | 128 [118-141.8] | 128 [118-138.3] | 0.278 |
| DBL (mmHg) | 72.5 [67-82] | 74 [67.8-80] | 0.817 |
| HDL-C (mg/dL) | 58 [47-70.5] | 57 [49-68.5] | 0.601 |
| Triglycerides (mg/dL) | 86 [59-122] | 88 [64-122] | 0.796 |
| FPG (mg/dL) | 96 [89-102] | 95 [90-102] | 0.820 |
| HbA1c (%) | 5.5 [5.3-5.6] | 5.5 [5.3-5.7] | 0.317 |
| hsCRP (mg/dL) | 1.5 [0.6-5.1] | 1.9 [0.7-3.3] | 0.335 |
| CVLT IR (score) | 63 [56-70] | 65 [60.5-72] | 1.8x10⁻⁴ |
| CVLT SDFR (score) | 14 [12-16] | 15 [13.5-16] | 0.005 |
| Total Digit Span (score) | 15 [12-17] | 15 [12.5-17] | 0.169 |
| PHQ-9 (score) | 5 3-9 | 4 [2-8] | 0.209 |

Not only was the microbiota composition associated with memory, but also the metagenome functions were linked to this cognitive trait (Figure 1e-f, Figure 5c). Bacterial functions related to vitamin B metabolism, particularly those involved in the one-carbon metabolism such as riboflavin (*ribBA; aphaA; fre, ubiB*)*,* vitamin B6 (*pdxA, epd*), folic acid (*pabB, queE, pabC, folM, folKP, folX*), and vitamin B12 (*ABC. VB12.P, btuC*), were negatively associated with all memory domains (highlighted in black in Figure 1e-f, Figure 5). There is convincing data for the association between B vitamins and cognition. In particular, it is well known that thiamine and folate impact memory. Bacterial functions related to thiamine metabolism (*thiB, thiK, thiP, ABC.VB1X.P, ABC.VB1X.A*)*,* another B vitamin, were also associated with low memory scores. The hypothesis is that these functions would result in preferential uptake or catabolism of thiamine by intestinal bacteria, resulting in decreased thiamine uptake by the host. Concordantly, significantly low plasma thiamine levels were found in subjects with lower memory scores (34.5 (27.2-45.3) vs. 44.3 (32.3-64.6) ng/ml, P = 0.016). Other relevant metagenomic functions associated with several memory domains included those related to the aromatic amino acids (AAA) metabolism, one-carbon metabolism and endocannabinoid signalling (highlighted in Figure 1e-f, Figure 5c) and are further discussed below.
When the associations separately in obese and non-obese subjects (Figure 1g-n, Figure 5d-g) were evaluated, it was found that several *Prevotella* sp. were positively associated with verbal memory among non-obese subjects (Figure 1g, Figure 5d) while *Eubacterium* and *Anaerovibrio* sp. showed similar associations within obese subjects (Figure 1i, Figure 5e). Bacteria belonging to phylum Proteobacteria were similarly and negatively associated in subjects without and with obesity, but preferentially in the latter. Regarding working memory, were observed positive associations of Selenomonadaceae, *Ruminococcus sp. and Colinsella intestinalis* in non-obese subjects (Figure 1k) and *Eubacterium sp., Ruminococcus sp., Clostridium sp. 27_14 and Faecalibacterium sp. CAG:74* in obese subjects (Figure 1m). The associations of bacterial functions related to thiamine were more marked among subjects with obesity (Figure 1j,n) who have been described to be particularly susceptible to thiamine deficits.
In summary, several species of the phylum Firmicutes (genera *Clostridium, Ruminococcus, and Eubacterium or* family Selenomonadaceae), were associated positively with memory scores. The phyla Bacteroidetes, Fusobacteria and Proteobacteria contained species mainly presented negative associations with memory scores.

To the best knowledge of the inventors, there are no previous descriptions of gut microbiota linked to the different memory domains in humans. Current results are in line with those identifying a higher prevalence of Bacteroidetes in patients with mild cognitive impairment. Species of the Enterobacteriaceae family as *Citrobacter rodentium* (phylum Proteobacteria) were associated with impaired memory in acute stress. *Ruminococcus gnavus* and different Bacteroidetes and *Enterobacter* species were increased in subjects with insulin resistance and obesity and associated with a worse cognitive profile (current findings). On the contrary, species of the phylum Firmicutes belonging to class *Clostridiales* and genus *Roseburia* linked to higher memory score had a decreased relative abundance in subjects with type 2 diabetes. In mice, the combined administration of *Lactobacillus rhamnosus* and *L. helveticus* led to increased non-spatial memory, improving c-Fos expression in the hippocampus.

### Brain structure is also associated with the Gut microbiome and bacterial functions differentially in obese and nonobese subjects

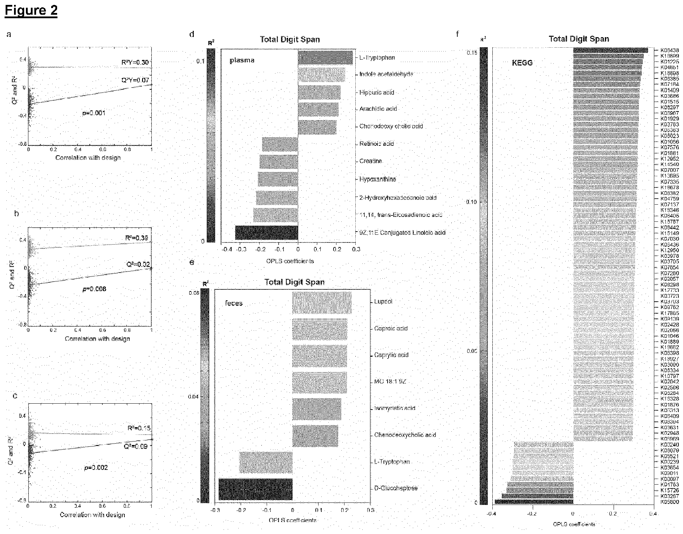

The volume of different brain areas involved in verbal and working memory in 143 subjects using magnetic resonance imaging were evaluated. Verbal and learning memory was associated with the volumes of the right and left hippocampus, and working memory with the right frontal inferior orbital (FIO) volume in all subjects after adjustment for age, BMI, sex, total intracranial volume (TIV) and PHQ-9 (from now on the term adjusted will refer to these adjustments) (Figure 2a). The hippocampal associations were also significantly and positive within non-obese subjects, although no significant associations were found with the frontal areas (Figure 2b-d). Conversely, working memory (TDS) was positively associated with the left FIO volume in all subjects (Figure 2a) and with other frontal areas within non-obese subjects (Figure 2b, Figure 2e-f). Notably, no significant associations among these memory domains and brain volumes were observed in obese individuals. The adjusted relationships between the baseline verbal and learning memory (free retrieval of words in CVLT tests) and the volumes of the right and left hippocampus as assessed one year later in 93 of the participants were also significant. These findings highlight different brain structures involved in verbal and working memory and are in line with previous reports linking verbal memory performance with prefrontal and temporal brain features, such as the hippocampus. Interestingly, several *Roseburia sp.* were found positively associated with verbal memory that were directly associated with the adjusted volume of the left hippocampus, and also concordant negative associations among *Bacteroides sp.,* verbal memory scores and the adjusted volume of left hippocampus (Figure 2g). Other concordant associations are shown in bold in Figure 2g.

On the other hand, *Acetitomaculum ruminis* was concomitantly associated with working memory and the adjusted volume of the right FIO area (Figure 2h) while several *Bacteroides sp.* appeared negatively and concordantly associated with both verbal and working memory, and the adjusted volume of both the left hippocampus and right FIO area (Figure 2h). Several bacterial functions concordantly associated with memory scores and adjusted volumes (both positively and negatively) were also found, shown in bold in Figure 2i. Of note, a function related to thiamine metabolism was associated with the adjusted right FIO volume.

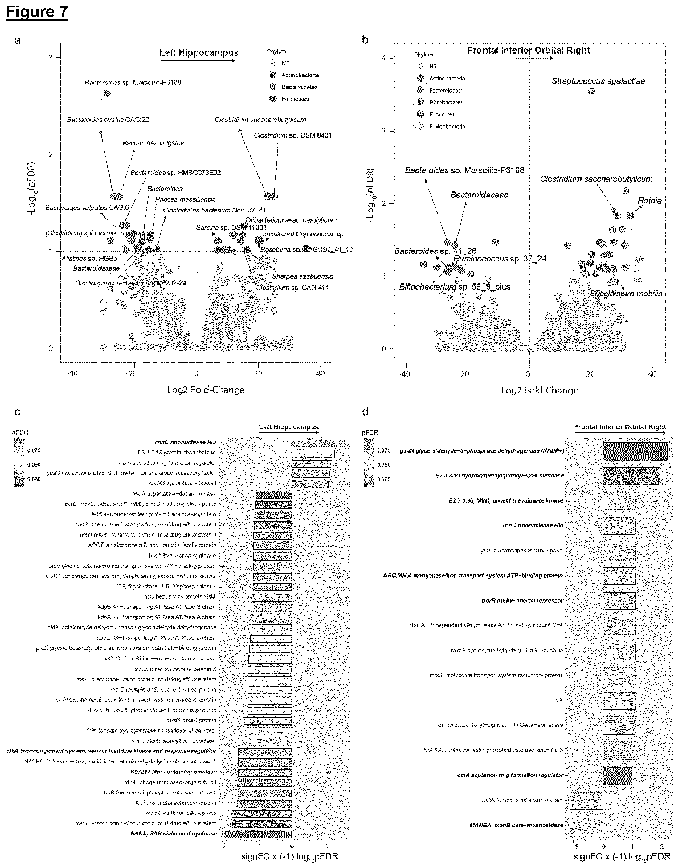

Notably, the metagenomic functions found to be associated with the volume of the hippocampus were also associated with verbal memory, while those associated with the FIO volume were also concordantly linked to working memory. In addition, the bacterial taxonomy and metagenomic functions were associated with the volume of brain areas and memory domains not only at baseline but also at follow up (Figure 7a-d).

When obese and non-obese subjects were evaluated separately, several bacterial functions that were found to be significantly linked with verbal and working memory, were also associated with the adjusted left hippocampus (Figure 2g) and right FIO volumes (Figure 2l), respectively in non-obese subjects (shown in bold). Remarkably, no associations were found among metagenomics functions and these brain volumes in obese subjects, which is in line with the lack of significant associations observed in obese patients among memory tests and selected brain volumes.

### Memory scores are differentially linked to plasma and faecal metabolomics and specific bacterial functions in obese and non-obese subjects

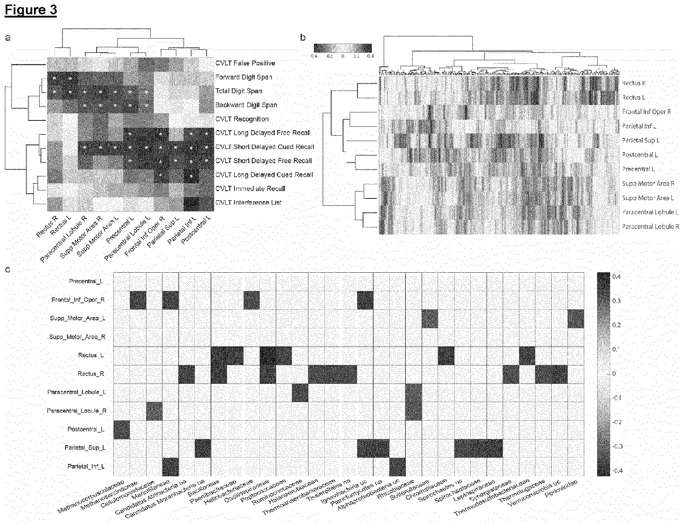

Metabolome-wide association studies (MWAS) using random forest-based machine learning variable selection techniques to identify plasma were then performed (Figure 3a-h, Figure 8a-h, Figure 9a-h) and faecal (Figure 3i-p, Figure 8i-p, Figure 9i-p) metabolites associated with the memory tests. Remarkably, the scores of all memory domains were associated with altered plasma levels of the aromatic amino acids (AAA) tryptophan, tyrosine and phenylalanine and their catabolites (Tryptophan catabolites: Indole-3-acetaldehyde (3-IAAId), Indole-3-propionic acid (3-IPA); Tyrosine catabolites: 4-hydroxyphenyllactic acid (4-HPLA), Phenylalanine catabolites: Phenylacetylglutamine, Phenylacetylglycine). These AAA are the precursor amino acids of serotonin and dopamine, two neurotransmitters that play a key role in the central nervous systems. Brain regions implicated in cognition such as the hippocampus and the PFC are vastly innervated by dopaminergic and serotonergic afferents and alterations in the serotonergic and dopaminergic neurotransmission are both associated with impaired learning and memory. Both tryptophan and tyrosine had positive associations with memory scores. This finding is in line with oral administration of tryptophan leading to improved memory acquisition, consolidation and storage in rodents.

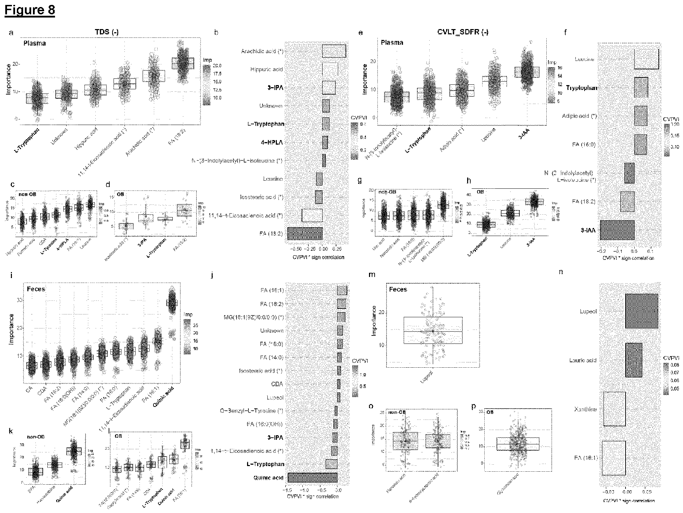

Previous studies have shown that antibiotic-induced alterations of the microbiota decreased both AAA concentrations and serotonin and dopamine levels in the porcine hypothalamus. The gut microbiota has also shown to directly metabolize tryptophan into several indole derivatives, which are potent ligands of the aryl hydrocarbon receptor (AhR). Deletion of the AhR has shown to alter adult hippocampal neurogenesis and contextual fear memory. Consistently, several indole derivatives positively associated with memory scores were found. In addition, several bacterial functions involved in tryptophan and phenylalanine metabolism negatively associated to the different memory domains were also identified (Figure 5h-i). In particular, functions related to tryptophan transporters such as tryptophan-specific transporter (*mtr*) and low affinity tryptophan permease (*tnaB*) had negative associations with the CVLT-SDFR. Quinate dehydrogenase (*quiA*), involved in tryptophan, tyrosine and phenylalanine metabolism had the strongest negative association with CVLT. Notably, faecal quinic acid had by far the strongest negative association with the TDS scores, followed by tryptophan (Figure 8i-j). The negative association between faecal tryptophan and memory scores might be related either to its transformation into tryptophan metabolites (see below) or to its increased systemic absorption.

Interestingly, the memory-related alterations in tryptophan metabolism were only observed in obese individuals, which is in line with the observed associations of tryptophan-related metagenomic functions and memory domains in obese, but not in non-obese patients. Chronic low-grade inflammation is a hallmark of obesity and the association between obesity and cognitive decline has recently been shown to be mediated by inflammation. Consistently, a strong positive association between BMI and hs-CRP (R=0.71, *P*<1*x*10⁻¹⁶) was found. Notably, more than 90% of tryptophan is metabolized through the kynurenine pathway, which is activated under inflammatory conditions. In line with this, plasma tryptophan levels had a negative correlation with the hs-CRP (R=-0.34, *P*<3.3*x*10⁻⁴). Importantly, microbial-derived products, including indoles, play a key role in the activation of indole-amine 2,3-dioxygenase (IDO), the rate limiting enzyme in the kynurenine pathway. There is previous evidence that these metabolites have an effect on astrocytes to limit inflammation of the central nervous system in experimental models. The current observations are the first in humans, to the best knowledge of the inventors, linking tryptophan and its metabolites to cognition. Cholinergic systems have also been linked to cognitive processes such as attention and memory. Hence, choline is the precursor of the neurotransmitter acetylcholine, but it can also be metabolized to betaine, a key methyl donor in the one-carbon metabolism and modulator of homocysteine status, whose elevated plasma levels have been implicated in learning and memory deficits. Thus, betaine supplementation has shown to prevent homocysteine-induced memory impairment via changes in the activity of MMP-9 in the frontal cortex. In agreement, circulating betaine levels associated with memory scores were found. These alterations in betaine levels are also in line with the associations between cognitive domains and several metagenomic functions involved in choline and betaine transporters, such as choline/betaine transport protein (betT,betS), betaine/proline transport systems ATP-binding protein (proV), betaine/proline transport systems substrate-binding protein (proX). Additionally, one of the functions most associated with short and immediate memory implicated the choline dehydrogenase (betA) gene, responsible for the conversion of choline to betaine. Interestingly, several alterations in metagenomic functions related to the metabolism of B vitamins involved in one-carbon metabolism, homocysteine levels and cognition were also found, mainly B2, B6, B9 and B12.

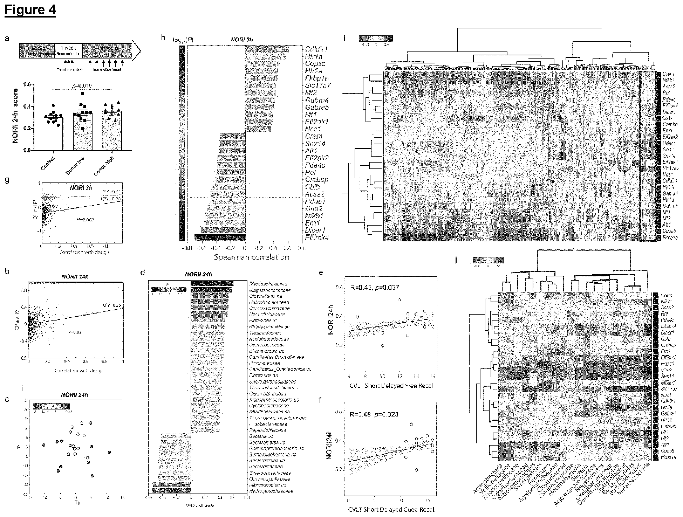

Other metabolites that had positive associations with the different memory domains were the endocannabinoids oleamide and arachidonoylethanolamide (AEA, anandamide). The endocannabinoids are lipid-derived mediators that play a key role in neurotransmission. Consequently, extensive evidence indicates a role of the endocannabinoid system in the modulation of cognition, particularly in learning and memory functioning. Anandamide has been reported to reverse hippocampal damage and memory impairment in rodents and protect neurons from amyloid-b cytotoxic effects. Similarly, oleamide administration significantly reversed memory and cognitive impairment in mice. Interestingly, one of the microbiota functions with the strongest associations with the cognitive domains in both humans and mice was the N-acetyl Phosphatidylethanolamine Phospholipase D (NAPEPLD) (Figure 1f, Figure 4h), which is necessary for the biosynthesis of fatty acid ethanol amides, including the endocannabinoids.

### Effects of microbiota transplantation from humans to mice.

The possible effects of the microbiota on memory scores in mice were then tested. The mouse behavioural models used in this study evaluated two different memory tasks. The cue-induced fear conditioning is a well-recognized model of emotional memories, whereas the novel object recognition paradigm is a widely used model of memories with a different neurobiological substrate. Indeed, cue-induced fear conditioning evaluates an associative learning to aversive stimuli, which behavioural responses are mainly mediated by the amygdala. Thus, the acquisition of fear conditioning learning is mediated by an association of the conditioned and unconditioned stimuli with the formation of memory traces of the conditioned cue within the basolateral amygdala. The subsequent presentation of the cue retrieves the memory trace and initiates a conditioned response, freezing, driven by the central amygdala. In contrast, the hippocampus plays a crucial role in the memory responses evaluated in the novel object recognition paradigm. The long-term memory traces evaluated in this paradigm are related to spatial memories not related to emotional aspects.
The novel object recognition cognitive task was performed using a V maze since the accuracy and reliability of the behavioural response is improved when compared to the use of an open-field for this task. Indeed, the exploration of the mouse is directed to the two different objects located in the extremes of the V maze, which improves their exploration and the discrimination index with a robust learning triggered by a single trial.

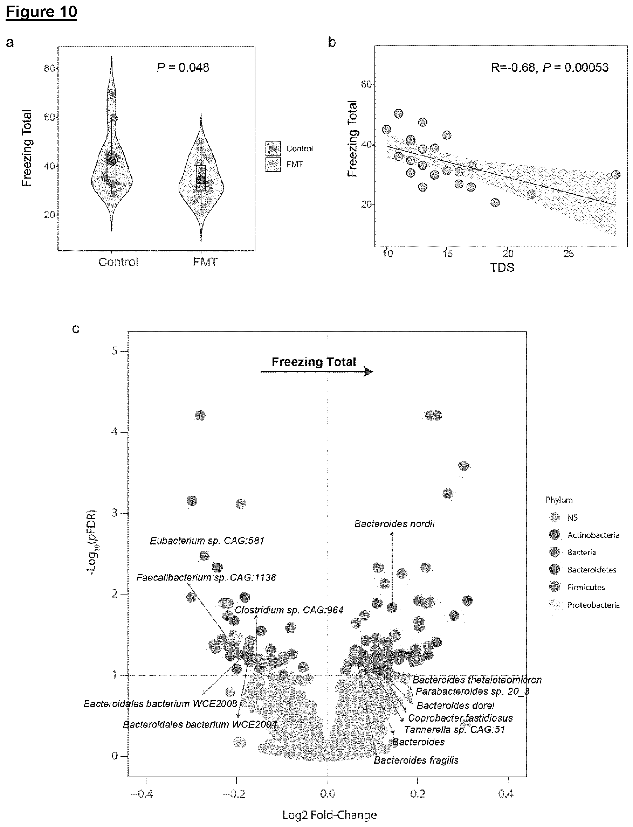

Microbiota from 22 subjects (11 with low and 11 with high memory scores matched for age, sex, BMI, and PHQ-9 scores) was orally delivered to individual mice in a blinded fashion (the investigator who performed the experiment was blinded regarding the origin of faeces). The effects on memory were compared with those of saline in 11 control mice. All mice were pre-treated with antibiotics for 14 days (Figure 4a). Mice receiving faecal microbiota transplantation (FMT) had higher scores in the Novel Object Recognition test at 24h (NORII24h) and lower Freezing Total scores than control mice (Figure 4b, Figure 10a). Interestingly, microbiota from non-obese donors led to significantly increased NORII24h scores compared with both obese donors (P = 0.026) and control mice (P = 0.009) (Figure 4c). Of note, both donor's CVLT-SDFR and CVLT-Short Delayed Cued Recall scores were significantly correlated with NORII24h scores in recipient's mice (Figure 4 d,e). Bacterial species from the donor's microbiota, including *Akkermansia sp.* and *Subdoligranulum sp.* (NORI 3h) (Figure 4f), and also different *Clostridium, Ruminococcus and Roseburia sp.* (NORI 24h) (Figure 4g) were associated with increased memory scores of recipient's mice, while several *Bacteroides sp.* were negatively associated. Accordingly, the same *Bacteroides* sp. were positively associated with the Freezing total scores (Figure 10c). Notably, several donor's metagenomics functions associated with the donor's TDS memory domains were also associated with the recipient's mice NORII24h scores, including the *NAPEPLD* (Figure 4h). Also, in line with the human results, other associated functions included those related to vitamin B6 (*pdxJ, pdxB*), B12 (*btuB*), and tryptophan metabolism (*trpA, trpB*).

Finally, an RNA sequencing of the prefrontal cortex (PFC) highlighted several significant genes associated to the NORI3h (Figure 4i). Notably, the gene with the highest negative fold change was that encoding for transthyretin (*ttr*)*,* which altered hippocampal expression has been associated with memory deficits in aged animals. The gene with the second strongest fold change was *slc6a3,* which encodes a dopamine transporter. It is also noticeable the direct association of NORI3h with the 5HT receptor genes *htr1a* and *htr2a* and the folate receptor gene *folr1,* which further emphasizes the links shown above between aromatic amino acids, folate metabolism and memory. The nuclear factor gene *nfkb1,* known to be crucial in the inflammatory cascade and in memory consolidation, was also directly associated to short-term memory; whereas *dicer1,* which knockout in mice enhanced memory, was negatively associated with this memory trait. Finally, *acss2* and *hdac1* were directly associated to short-term memory, confirming recent observations of brain histone acetylation relationships with associative learning. Interestingly, the expression of the memory genes associated to the NORI3h was simultaneously associated with different bacterial clusters and in the same direction (Figure 4j).
To sum up, current findings point at the existence of an ecosystem of bacteria that are simultaneously linked to verbal and working memory, the volume of brain areas involved in these traits and with plasma/faecal tryptophan, microbiota-driven tryptophan metabolites and 5HT receptors expression in the PFC. Several of the species identified here have been previously linked positively (*Roseburia, Subdoligranulum, Faecalibacterium*) and negatively (*Fusobacterium, Bacteroides*) to healthy eating scores in the same direction that that to increased and decreased memory scores described here. The gut microbiota phenocopied different memory traits from humans to mice.

### FIGURES

**Figure 1****. A characteristic microbiota taxonomic and functional profile is associated with memory scores and modulated by obesity.**
   **a)** Boxplot for the total digit span (TDS) and
   **b)** California Verbal Learning Short Delayed Free Recall (CVLT_SDFR) in non-obese and obese patients. Differences between groups were analysed by a Wilcoxon tests.
   **c)** Volcano plots of differential bacterial abundance associated with the CVLT_SDFR and
   **d)** the TDS, as calculated by DESeq2 from shotgun metagenomic sequencing in the IRONMET cohort, adjusting for age, BMI, sex, education years, and Patient Health Questionnaire (PHQ)-9 scores. Fold change associated with a unit change in the corresponding test and Benjamini-Hochberg adjusted p-values (*p*FDR) are plotted for each taxon. Significantly different taxa are coloured according to phylum.
   **e)** Manhattan-like plot of significantly expressed KEGG bacterial genes associated with the CVLT_SDFR (*p*FDR <0.002) and
   **f)** TDS (*p*FDR <0.04), identified from DESeq2 analysis adjusted for age, BMI, sex, educations years, and PHQ-9. The -log₁₀(*p*FDR) values are multiplied by the fold change (FC) sign to take into account the direction of the association. Bars are coloured according to the pFDR. Those functions related to B vitamins metabolism, one-carbon metabolism, phenylalanine, tryptophan, and endocannabinoid metabolism are highlighted in black.
   **g-n)** Taxonomic and functional associations for the CVLT_SDFR and TDS tests in non-obese and obese patients.
**Figure 2****. The gut microbiota is associated with brain structure.**
   **a)** Heatmap showing the partial correlations (adjusted by age, sex, BMI, education years, PHQ-9 and total intracranial volume (TIV)) between the TDS and CVLT_SDFR tests and selected brain volumes in all (obese and non-obese) patients and
   **b)** in non-obese patients. Significant associations are shown with a cross: +, *p* < 0.05; ++ *p* < 0.01. No significant associations were found in obese individuals and are not shown.
   **c,d)** After controlling for the above covariates, the left hippocampus volume had a positive association with the CVLT_SDFR, whereas the
   **h,i)** Right frontal inferior orbital volume was positively associated with the TDS. Both associations were more marked when only non-obese individuals were considered.
   **e)** Volcano plots of differential bacterial abundance associated with the left hippocampus volume and
   **j)** right frontal inferior orbital volume, as calculated by DESeq2 controlling for covariates. Fold change associated with a unit change in the corresponding volumes and Benjamini-Hochberg adjusted *p*-values (*p*FDR) are plotted for each taxon. Significantly different taxa are coloured according to phylum. Taxa that were also associated with the memory domains are highlighted in bold.
   **f)** Manhattan-like plot of significantly expressed KEGG bacterial genes associated with the left hippocampus volume and
   **k)** the right frontal inferior orbital volume, identified from covariate-adjusted DESeq2 analysis. The -log₁₀(pFDR) values are multiplied by the fold change (FC) sign to take into account the direction of the association. Bars are coloured according to the pFDR. Metagenomic functions that were also associated with the several cognitive domains are highlighted in bold.
   **g)** The results of the same functional analysis for the left hippocampus volume and **I)** right frontal inferior orbital volume in non-obese individuals. No significant functional associations were found in obese patients for these brain volumes.
**Figure 3****. Plasma and faecal metabolomics in electrospray ionization (ESI) positive mode linked to memory domains.**
   **a)** Boxplots of the normalized permutation importance measure for the metabolites associated to the to the CVLT_SDFR in plasma,
   **e)** the TDS in plasma,
   **i)** the CVLT_SDFR in faeces, and
   **m)** the TDS in faeces, identified by machine learning thorough the random forest-based Boruta feature selection algorithm at each of the 500 iterations.
   **b)** Cross-validated permutation variable importance (CVPVI) measure × sign of the correlation between each metabolite associated to the CVLT_SDFR test in plasma,
   **f)** the TDS in plasma,
   **j)** the CVLT_SDFR in faeces, and
   **n)** the TDS in faeces, identified by machine learning using the random forest-based Vita method.
   **c-d)** Normalized permutation importance measure for Boruta selected metabolites associated to the CVLT_SDFR in plasma,
   **g-h)** the TDS in plasma,
   **k-l)** the CVLT_SDFR in plasma, and
   **o-p)** the TDS in faeces, in non-obese and obese patients, respectively. All metabolites were identified based on exact mass, retention time and MS/MS spectrum, except those with (*) that were only identified based on exact mass and retention time. 3-IAAId, Indole-3-acetaldehyde; AEA, arachidonoylethanolamide; CA, cholic acid; CDA, chenodeoxycholic acid; FA, fatty acid.
**Figure 4****. Human donor's and recipient's mice memory became aligned through the microbiota.**
   **a)** Experimental design for the faecal microbiota transplantation (FMT) study. The microbiota from low-memory (*n*=11) and high-memory (*n*=11) human donors was delivered to recipient mice pre-treated with antibiotics for 14 days. *n* =11 control mice were treated with saline. Cognitive tests were performed after 4 weeks.
   **b)** Violin plots for the Novel Object Recognition tests comparing the control group and the FMT group (t-test), and
   **c)** comparing the control group to the groups receiving microbiota from non-obese and obese human donors (one-way ANOVA).
   **d,e)** Spearman correlation between the California Verbal Learning (CVLT) tests in humans and the NORII24h in mice.
   **f)** Volcano plots of differential human donor bacterial abundance associated with the recipient's mice NORI3h and
   **g)** the NORII24h, from DESeq2 analysis. Fold change associated with a unit change in the corresponding memory test and Benjamini-Hochberg adjusted *p*-values (*p*FDR) are plotted for each taxon. Significantly different taxa are coloured according to phylum.
   **h)** Manhattan-like plot showing only the significantly expressed KEGG bacterial genes associated with the mice NORII24h test (pFDR <0.05) that were also associated to the total digit span score in humans. The -log₁₀(pFDR) values are multiplied by the fold change (FC) sign to take into account the direction of the association. Bars are coloured according to the pFDR.
   **i)** Volcano plot of differential prefrontal cortex (PFC) genes associated with the NORI3h. Fold change associated with a unit change in the NORI3h test and Benjamini-Hochberg adjusted *p*-values (*p*FDR) are plotted for each gene. Those genes with the highest FC and the lowest *p*FDR values are highlighted. Genes with a possible role in memory based on the literature are also highlighted.
   **j)** Correlation heatmap among mice bacterial species and selected PFC genes associated with NORI3h. Clustering was performed using Euclidean distances and Ward linkage. Three bacterial clusters with strong correlations were identified and highlighted. These involve bacterial species positively linked to both the NORI3h and PFC genes positively associated with the NORI3h, and bacterial species negatively associated to the NORI3h and at the same negatively associated to PFC genes positively associated with the NORI3h and positively associated to genes negatively associated with the NORI3h.
**Figure 5****. Microbiota taxonomic and functional profiles associated to the California Verbal Learning Immediate Recall (CVLT_IR).**
   **a)** Boxplot for the CVLT_IR in non-obese and obese patients. Differences between groups were analysed by a Wilcoxon tests.
   **b)** Volcano plots of differential bacterial abundance associated with the CVLT_IR calculated by DESeq2 controlling for age, BMI, sex, education years, and Patient Health Questionnaire (PHQ)-9 scores. Fold change associated with a unit change in the CVLT_IR and Benjamini-Hochberg adjusted *p*-values (*p*FDR) are plotted for each taxon. Significantly different taxa are coloured according to phylum.
   **c)** Manhattan-like plot of significantly expressed KEGG bacterial genes associated with the CVLT_IR (*p*FDR <0.005) identified from DESeq2 controlled for the previous covariates. The -logio(*p*FDR) values are multiplied by the fold change (FC) sign to take into account the direction of the association. Bars are coloured according to the *p*FDR. Those functions related to B vitamins metabolism, one-carbon metabolism, phenylalanine, tryptophan, and endocannabinoid metabolism are highlighted in black. The complete list of significantly associated metagenomic functions can be found in Table S1.
   **d-g)** Taxonomic and functional associations for the CVLT_IR in non-obese and obese patients.
   **h)** Manhattan-like plot of significantly expressed KEGG bacterial genes related to the phenylalanine metabolism and
   **i)** the tryptophan metabolism associated with the CVLT Short Delayed Free Recall.
**Figure 6****. Microbiota composition associated with memory domains in the replication longitudinal cohort.**
   **a)** Volcano plots of differential bacterial abundance associated with the California Verbal Learning Tests Short Delayed Free Recall (CVLT_SDFR),
   **b)** the CVLT Immediate Recall (IR), and
   **c)** the Total Digit Span (TDS) in 69 consecutive subjects after 1 year of follow-up as calculated by DESeq2 analysis controlling for age, body mass index, sex, education years, and Patient Health Questionnaire (PHQ)-9 scores. Fold change associated with a unit change in the corresponding test and Benjamini-Hochberg-adjusted *p*-values (*p*FDR) are plotted for each taxon. Significantly different taxa are coloured according to phylum. Only the bacterial species that were also significantly associated with the memory domains at baseline and in the same direction are highlighted.
**Figure 7****. Microbiota taxonomic and functional associations the brain structure in the replication longitudinal cohort.**
   **a)** Volcano plots of differential bacterial abundance associated with the left hippocampus volume and
   **b)** right frontal inferior orbital volume in the replication cohort after 1 year of follow-up, as calculated by DESeq2 controlling for age, BMI, sex, education years, PHQ-9 and total intracranial volume (TIV). Fold change associated with a unit change in the corresponding volumes and Benjamini-Hochberg adjusted p-values (pFDR) are plotted for each taxon. Significantly different taxa are coloured according to phylum. Only those bacterial species that were also significantly associated with the corresponding brain volumes at baseline and in the same direction are highlighted.
   **c)** Manhattan-like plot of significantly expressed KEGG bacterial genes associated with the left hippocampus volume and
   **k)** the right frontal inferior orbital in the replication cohort after 1 year of follow-up, identified from covariate-adjusted DESeq2 analysis. For the left hippocampus, only those bacterial functions also associated to the memory domains are represented. The - logio(pFDR) values are multiplied by the fold change (FC) sign to take into account the direction of the association. Bars are coloured according to the pFDR. Metagenomic functions that were also associated with the corresponding brain volumes at baseline are highlighted in bold. The complete list of significantly associated functions can be found in Supplemental Table S3g-j.
**Figure 8****. Plasma and faecal metabolomics in electrospray ionization (ESI) negative mode linked to memory domains.**
   **a)** Boxplots of the normalized permutation importance measure for the metabolites associated to the to the TDS in plasma,
   **e)** the CVLT_SDFR in plasma,
   **i)** the TDS in faeces, and
   **m)** the CVLT_SDFR in faeces, identified by machine learning thorough the random forest-based Boruta feature selection algorithm at each of the 500 iterations.
   **b)** Cross-validated permutation variable importance (CVPVI) measure × sign of the correlation between each metabolite associated to the TDS test in plasma,
   **f)** the CVLT_SDFR in plasma,
   **j)** the TDS in faeces, and
   **n)** the CVLT_SDFR in faeces, identified by machine learning using the random forest-based Vita method.
   **c-d)** Normalized permutation importance measure for Boruta selected metabolites associated to the TDS in plasma,
   **g-h)** the CVLT_SDFR in plasma,
   **k-l)** the TDS in plasma, and
   **o-p)** the CVLT_SDFR in faeces. All metabolites were identified based on exact mass, retention time and MS/MS spectrum, except those with (*) that were only identified based on exact mass and retention time. 3-IPA, Indole-3-propionic acid; CA, cholic acid; CDA, chenodeoxycholic acid; FA, fatty acid; 4-HPLA, 4-hydroxyphenyllactic acid; MG, monoglyceride.

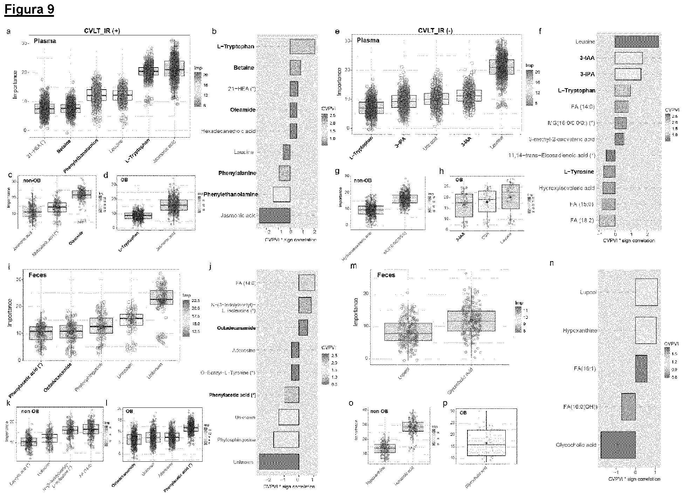

**Figure 9****. Plasma and faecal metabolomics linked to the California Verbal Learning Tests Immediate Recall (CVLT_IR).**
   **a)** Boxplots of the normalized permutation importance measure for the metabolites associated to the to the CVLT_IR in positive ESI mode in plasma,
   **e)** negative ESI mode in plasma,
   **i)** positive ESI mode in faeces, and
   **m)** negative ESI mode in faeces, identified by machine learning thorough the random forest-based Boruta feature selection algorithm at each of the 500 iterations.
   **b)** Cross-validated permutation variable importance (CVPVI) measure × sign of the correlation between each metabolite associated to the CVLT_IR in ESI positive mode in plasma,
   **f)** negative ESI mode in plasma,
   **j)** positive ESI mode in faeces, and
   **n)** negative ESI model in faeces, identified by machine learning using the random forest-based Vita method.
   **c-d)** Normalized permutation importance measure for Boruta selected metabolites associated to the CVLT_IR in positive ESI mode in,
   **g-h)** negative ESI mode in plasma,
   **k-l)** positive ESI model in faeces, and
   **o-p)** negative ESI model in faeces, for non-obese and obese patients, respectively. All metabolites were identified based on exact mass, retention time and MS/MS spectrum, except those with (*) that were only identified based on exact mass and retention time. 21-HEA, 21-hydroxyheneicosanoic acid; 3-IAA, Indoe-3-acetic acid; 3-IPA, Indole-3-propionic acid; CA, cholic acid; CDA, chenodeoxycholic acid; FA, fatty acid; MG, monoglyceride.
**Figure 10****. Results for the freezing total test in the faecal microbiota transplantation (FMT) experiment.**
   **a)** Violin plots for the Freezing total test scores comparing the control group and the FMT group (t-test).
   **b)** Spearman correlation between the freezing total test in recipient's mice and the total digit span (TDS) test in human donors.
   **c)** Volcano plots of differential human donor bacterial abundance associated with the recipient's mice freezing total test from DESeq2 analysis. Fold change associated with a unit change in the freezing total test and Benjamini-Hochberg adjusted *p*-values (pFDR) are plotted for each taxon. Significantly different taxa are coloured according to phylum. Only significant species that were also associated inversely in the NORII24h test are highlighted.

### EXAMPLES

### STAR Methods

### Example 1: Clinical study.

### Recruitment of study subjects:

From January 2016 to October 2017, a cross-sectional case-control study was undertaken in the Endocrinology Department of Josep Trueta University Hospital. Consecutive subjects with obesity (body mass index, BMI≥30kg/m²) and age- and sex-matched nonobese subjects (BMI 18.5-<30kg/m²) were included, with an age range of 27.2-66.6 years. Exclusion criteria were: type 2 diabetes mellitus, chronic inflammatory systemic diseases, acute or chronic infections in the previous month; use of antibiotic, antifungal, antiviral or treatment with proton-pump inhibitors; severe disorders of eating behaviour or major psychiatric antecedents; neurological diseases, history of trauma or injured brain, language disorders; and excessive alcohol intake (≥ 40 g OH/day in women or 80g OH/day in men). The Institutional review board - Ethics Committee and the Committee for Clinical Research (CEIC) of Dr. Josep Trueta University Hospital (Girona, Spain) approved the study protocol and informed written consent was obtained from all participants. *Clinical and laboratory parameters:* Body composition was assessed using a dual energy x-ray absorptiometry (DEXA, GE lunar, Madison, Wisconsin). Fasting plasma glucose (FPG), lipids profile and high-sensitivity C-reactive protein (hsCRP) levels were measured using an analyser (Cobas^{®} 8000 c702, Roche Diagnostics, Basel, Switzerland). Glycated haemoglobin (HbA1c) was determined by performance liquid chromatography (ADAM^{®}A1c HA-8180V, ARKRAY, Inc., Kyoto, Japan). *Dietary pattern:* The dietary characteristics of the subjects were collected in a personal interview using a validated food-frequency questionnaire (Vioque, J. et al. 2013. Reproducibility and validity of a food frequency questionnaire among pregnant women in a Mediterranean area. Nutr. J. 12, 26.)

### Example 2: MRI study.

*MRI acquisition and image pre-processing:* All subjects were studied on a 1.5T Ingenia (Philips Healthcare, Best, The Netherlands) with eight channel head coils. Structural images were acquired using a 3D Turbo Field Echo Planar Imaging (TFEPI) sequence and parameters of echo time (TE) = 4.1ms, repetition time (TR) = 8.4ms, flip angle 8°, field of view (FOV) 230x190 matrix. A total of 145 whole-brain images per subject with thickness axial slices of 1x1x1mm³ with or without gap. The total scan time was 189.6s. The anatomical imaging data was processed and analysed using MATLAB version R2017a (The MathWorks Inc, Natick, Mass) and Statistical Parametric software (SPM12; The Welcome Department of Imaging Neuroscience, London). Pre-processing steps involved motion correction, spatial normalization and smoothing using a Gaussian filter (FWHM 8 mm). Data were normalized to Diffeomorphic Anatomical Registration Through Exponentiated Lie (DARTEL) and resliced to a 2mm isotropic resolution in Montreal Neurological Institute (MNI) space. Volumetric brain analyses: The Automated Anatomical Labelling (AAL) (Tzourio-Mazoyer, N. et al. 2002. Automated anatomical labelling of activations in SPM using a macroscopic anatomical parcellation of the MNI MRI single-subject brain. Neuroimage 15, 273-289) atlas was used to obtain the volumetric information of the right and left hippocampus, opercula (orbitalis, tringularis, opercularis), and middle and superior frontal gyri as informed by the involvement of these brain regions in verbal memory (Aslaksen, P.M. et al. 2018. The relation of hippocampal subfield volumes to verbal episodic memory measured by the California Verbal Learning Test II in healthy adults. Behav. Brain Res. 351, 131-137. Gross, L.A. etal. 2018. Neural correlates of verbal memory in youth with heavy prenatal alcohol exposure. Brain Imaging Behav. 12, 806-822. Yu, Q. et al. 2018. Age-associated increase in mnemonic strategy use is linked to prefrontal cortex development. Neuroimage 181, 162-169) in 14394 participants. Volumetric differences for these targeted regions between participants with and without obesity were explored using independent sample t-tests, and Pearson Partial correlations were used to explore for Each region was orthogonalized for sex, age and total grey matter volume in MATLAB version R2017a (The Math Works Inc, Natick, MA) and subsequently entered to SPSS to investigate associations between the grey matter volumes and the performance in the CVLT and the digit tasks controlling for age, sex, education, depressive symptoms, BMI and total intracranial volume in the whole sample, and within the obese and non-obese groups. Finally, the associations between the volume in the selected brain regions and the microbiota were investigated using Spearman correlation analyses corrected for multiple comparisons using q-values (Storey, J.D. 2002. A Direct Approach to False Discovery Rates. J. R. Stat. Soc. Ser. B (Statistical Methodol. 64, 479-498).

### Example 3: Neuropsychological assessment:

*California Verbal Learning Test-II (CVLT):* CVLT is used to assess verbal learning and memory (Delis, D.C. et al. 2000. Manual for the California Verbal Learning Test, (CVLT-II) (San Antonio, Texas: The Psychological Corporation). It consists of 5 learning tests in which a list of words (list A) is presented and the subject is asked, immediately after each presentation, to recall as much words as possible. Then an interference list (list B) is presented, and the subject is asked to repeat the same task. CVLT Immediate Recall score is a result of the first five tests and provides information about the learning process. In the short delay test, the patient is asked to recall list A, free (CVLT Short Delayed Free Recall) or with semantic facilitation (CVLT Short Delayed Cued Recall). A higher score reflects a better memory function. About 30 minutes are necessary to administrate this test and its reliability ranges from 0.78-0.94. *Digit Span:* Working memory was assessed by the Digit Span, a subtest of the Wechsler Adult Intelligence Scale-III (WAIS-III) (Wechsler, D. 2012. WAIS-IV. Escala de inteligencia de Wechsler para adultos-IV. Manual técnico y de interpretación (Madrid: NCS Pearson) a measure of general intellectual function. It is based on numbers and includes the Forward and Backward Digit Span tests. In the Forward Digit Span test, the examinee repeats a number sequence in the same order as presented. This constitutes a measure of working memory but also of attention. In the Backward Digit Span, the examinee repeats the number sequence in reverse order. Total Digit Span represents the total score of the two previous tests. A higher score reflects a better memory function. In a standardization sample of 394 participants (aged 16-89 years), the reliability coefficient was very high, ranging from 0.94-0.97 (Strauss, E. et al. 2006. A Compendium of Neuropsychological Tests: Administration, Norms, and Commentary (New York: Oxford University Press). *The Patient Health Questionnaire-9 (PHQ-9):* is a depression module of the PRIME-MD diagnostic instrument for mental disorders (Spitzer, R.L. et al. 1999. Validation and utility of a self-report version of PRIME-MD: the PHQ primary care study. Primary Care Evaluation of Mental Disorders. Patient Health Questionnaire. JAMA 282, 1737-1744). It encompasses 9 items of depression symptoms plus a question about functional impairment and can be scored as a depression severity rating (scores of 10-14 moderate, 15-19 moderately severe and 20-27 severe depressive symptoms) or with an algorithm based on the DSM-IV criteria (major and minor episode). Scores of 10 or more have an 88% sensitivity and specificity. PHQ-9 score was considered as a possible confounding factor in the analyses.

**Longitudinal cohort:** Cognitive tests and MRI variables were collected again in 93 consecutive subjects after 1 year of follow up.

### Example 4: Extraction of faecal genomic DNA and whole-genome shotgun sequencing

Total DNA was extracted from frozen human stools using the QIAamp DNA mini stool kit (Qiagen, Courtaboeuf, France). Quantification of DNA was performed with a Qubit 3.0 fluorometer (Thermo Fisher Scientific, Carlsbad, CA, USA), and 1 ng of each sample (0.2 ng/µl) was used for shot gun library preparation for high-throughput sequencing, using the Nextera DNA Flex Library Prep kit (Illumina, Inc., San Diego, CA, USA) according to the manufacturers' protocol. Sequencing was carried out on a NextSeq 500 sequencing system (Illumina) with 2 X 150-bp paired-end chemistry, at the facilities of the Sequencing and Bioinformatic Service of the FISABIO (Valencia, Spain). The obtained input fastq files were decompressed, filtered and 3. ends-trimmed by quality, using prinseq-lite-0.20.4 program (Schmieder, R. and Edwards, R. 2011. Quality control and pre-processing of metagenomic datasets. Bioinformatics 27, 863-864) and overlapping pairs were joined using FLASH-1.2.11 (Magoč, T., and Salzberg, S.L. 2011. FLASH: Fast length adjustment of short reads to improve genome assemblies. Bioinformatics 27, 2957-2963). Fastq files were then converted into fast files, and human and mouse host reads were removed by mapping the reads against the GRCh38.p11, reference human genome, and GRCm38.p6, reference mouse genome, respectively, by using bowtie2-2.3.4.3 (Langmead, B., and Salzberg, S.L. 2012. Fast gapped-read alignment with Bowtie 2. Nat. Methods 9, 357-359) with end-to-end and very sensitive options. Next, functional analyses were carried out by assembling the non-host reads into contigs by MEGAHIT v1.1.2 (Li, D., Liu, C.M., Luo, R., Sadakane, K., and Lam, T.W. 2015. MEGAHIT: An ultra-fast single-node solution for large and complex metagenomics assembly via succinct de Bruijn graph. Bioinformatics 31, 1674-1676) and mapping those reads against the contigs with bowtie2. Reads that did not assemble were appended to the contigs. Next, the program Prodigal v2.6.342 (Hyatt, D. et al. 2010. Prodigal: Prokaryotic gene recognition and translation initiation site identification. BMC Bioinformatics 11. 119) was used for predicting codifying regions. Functional annotation was carried out with HMMER (Durbin, R. et al. 1998. Biological Sequence Analysis: Probabilistic Models of Proteins and Nucleic Acids (Cambridge, UK) against the Kyoto Encyclopaedia of Genes and Genomes (KEGG) database, version 2016 (Kanehisa, M. 2000. KEGG: Kyoto Encyclopaedia of Genes and Genomes. Nucleic Acids Res. 28, 27-30) to obtain the functional subcategory, route and annotation of the genes. The filtering of the best annotations and the assignment of the orf annotation to every read were carried out using the statistical package R 3.1.0 (R Development Core Team 2013. R: A Language and Environment for Statistical Computing (Vienna, Austria) which also was used to count the aligned reads and to add the category and its coverage, and finally to build abundance matrices. Taxonomic annotation, was implemented with Kaiju v1.6.2 (Menzel, P. et al. 2016. Fast and sensitive taxonomic classification for metagenomics with Kaiju. Nat. Commun. 7, 11257) on the human and mouse-free reads. Addition of lineage information was added, counting of taxa and generation of an abundance matrix for all samples were performed using the package R. *Ratios of bacteria positively*/*negatively associated with memory:* As a proxy measure of memory, two ratios between RA of beneficial (those bacterial phyla and families associated with increased memory scores) and deleterious (bacterial phyla and families associated with decreased memory scores) were constructed. Ratio 1 contains the RA of Bifidobacteriaceae, Odoribacteraceae, Ruminococcaceae families in the numerator and RA of unclassified Enterobacterales, Enterobacteriaceae, Enterococcaceae, Pseumonadaceae, Actinomycetaceae families in the denominator. Ratio 2 contains RA of unassigned Anaerolineae, unassigned Blastocatellia, Clostridiaceae, unclassified Rhodospirillales, Sporolactobacillaceae in the numerator and RA of Alcaligenaceae and Erwiniaceae in the denominator.

### Example 5: Metabolomics analyses

For non-targeted metabolomics analysis, metabolites were extracted from faecal and plasma samples with methanol (containing phenylalanine-C13 as an internal standard) according to previously described methods (Wikoff et al., 2008; 5). Briefly, for plasma samples 30µl of cold methanol were added to 10 µl of each sample, vortexed for 1 minute and incubated for one hour at -20 °C. For faecal samples, the content of a 1.2 ml tube of Lysing Matrix E (MP biomedicals) and 600 µl of cold methanol were added to 10mg of sample. Samples were homogenized using FastPrep-24^{™} (MP biomedicals) and were incubated overnight in a rocker at 4°C. Then, all samples were centrifuged for three minutes at 12,000g, the supernatant was recovered and filtered with a 0.2 µm Eppendorf filter. Two µL of the extracted sample were applied onto a reversed-phase column (Zorbax SB-Aq 1.8 µm 2.1 x 50 mm; Agilent Technologies) equipped with a precolumn (Zorbax-SB-C8 Rapid Resolution Cartridge 2.1 x 30 mm 3.5 µm; Agilent Technologies) with a column temperature of 60°C. The flow rate was 0.6 mL/min. Solvent A was composed of water containing 0.2% acetic acid and solvent B was composed of methanol 0.2% acetic acid. The gradient started at 2% B and increased to 98% B in 13 min and held at 98% B for 6 min. Post-time was established in 5 min.

Data were collected in positive and negative electrospray modes time of flight operated in full-scan mode at 50-3000 m/z in an extended dynamic range (2 GHz), using N2 as the nebulizer gas (5 L/min, 350°C). The capillary voltage was 3500 V with a scan rate of 1 scan/s. The ESI source used a separate nebulizer for the continuous, low-level (10 L/min) introduction of reference mass compounds 121.050873 and 922.009798, which were used for continuous, online mass calibration. MassHunter Data Analysis Software (Agilent Technologies, Barcelona, Spain) was used to collect the results, and MassHunter Qualitative Analysis Software (Agilent Technologies, Barcelona, Spain) to obtain the molecular features of the samples, representing different, co-migrating ionic species of a given molecular entity using the Molecular Feature Extractor algorithm (Agilent Technologies, Barcelona, Spain), as described 5,6. Samples with a minimum of 2 ions were selected. Multiple charge states were forbidden. Compounds from different samples were aligned using a retention time window of 0.1% ± 0.25 minutes and a mass window of 20.0 ppm ±2.0 mDa. Only those present in at least 50% of the samples of one group and corrected for individual bias were selected.

### Example 6: Animal experiments

Male C57BL/6J mice (Charles River, France), weighing 23-26 g at the beginning of the experiment were used in this study. Mice were housed individually in controlled laboratory conditions with the temperature maintained at 21 ± 1°C and humidity at 55 ±10%. Animal procedures were conducted in strict accordance with the guidelines of the European Communities Directive 86/609/EEC regulating animal research and were approved by the local ethical committee (CEEA-PRBB). All the experiments were performed under blinded conditions (the researcher who administered the microbiota was blinded in relation to the memory scores of the subjects who provided the faeces). Mice were given a cocktail of ampicillin and metronidazole, vancomycin (all at 500 mg/L), ciprofloxacin HCI (200 mg/L), imipenem (250 mg/L) once daily for 14 consecutive days in drinking water, as previously described (Kelly, J.R. et al, 2016. Transferring the blues: Depression-associated gut microbiota induces neurobehavioral changes in the rat. J. Psychiatr. Res. 82, 109-118). Seventy-two hours later, animals were colonized via daily oral gavage of donor microbiota (150 µL) for 3 days. Animals were orally gavaged with saline (n=11) and faecal material from healthy volunteers' samples from humans with better cognitive scores (*n*=11) and humans with decreased cognitive scores (*n*=11)). No differences were found related to BMI, age, years, sex within these two groups. To offset potential confounder and/or cage effects and to reinforce the donor microbiota phenotype, booster inoculations were given twice per week throughout the study. Animals were exposed to a series of behavioural testing including novel object recognition (NOR) test and fear conditioning with nociception assessed by the hot plate test to ensure specificity.

The NOR was performed in a V-maze as previously published (Burokas, A. et al. 2014. Relationships between serotonergic and cannabinoid system in depressive-like behaviour: a PET study with [11C]-DASB. J. Neurochem. 130, 126-135). Three phases of 9-min were performed on consecutive days. Mice were first habituated to the V-maze. On the second day, 2 identical objects (chess pieces) were presented to the mice, and the time that they spent exploring each object was recorded. In the test phase (3h later for short-term memory or 24h later for long-term memory), 1 of the familiar objects was replaced with a novel object (a different chess piece), and the time spent exploring each object (novel and familiar) was computed. A discrimination index was calculated as the difference between the times that the animal spent exploring the novel (Tn) and familiar (Tf) object divided by the total time of object exploration: (Tn-Tf)/(Tn + Tf).

Fear conditioning conducted as described previously with some modifications (Saravia, R. et al. 2019. Concomitant THC and stress adolescent exposure induces impaired fear extinction and related neurobiological changes in adulthood. Neuropharmacology 144, 345-357). Mice were individually placed in a shuttle chamber (LE918, Panlab, Barcelona) surrounded by a sound-attenuating cabinet. The chamber floor was formed by parallel stainless-steel bars connected to a scrambled shock generator. On the training day, mice were habituated to the chamber during 180 s before the exposure to an acute beeping 30 s sound (80 dB). Each animal received an unconditioned stimulus (US) (0.6 mA footshock during 2 s) paired with the end of the sound (conditioned stimulus, CS). After the shock, the animal remained for 60 s in the shuttle chamber. To evaluate cued fear conditioning, mice were re-exposed to the CS in a novel environment (a wide white cylinder in the chamber) 24 h after the conditioning session. Mice were allowed to adapt for 180 s to the new environment which was followed by 30 s of the sound used in the training day. After the last sound trial, mice remained in the cylinder for 60 s. Fear memory was assessed as the percentage of time that mice spent freezing during the session. Freezing response, a rodent's natural response to fear, was evaluated by direct observation and defined as complete lack of movement, except for respiration for more than 1 s. The procedure was performed between 8.00 and 12.00 h in an experimental room different to the housing room.

At the end of the study the animals were consecutively sacrificed. The cecum was removed, weighted and stored, and the faeces collected and stored at -80 °C for further microbiota analysis. Faecal microbiota composition from mice was also analysed following the same procedures as humans.

### Example 7: Study of gene expression in prefrontal cortex

Sample preparation: The brains were quickly removed and the medial prefrontal cortex was dissected according to the atlas of stereotaxic coordinates of mouse brain (Paxinos, G. et al. 1997. The mouse brain in stereotaxic coordinates (San Diego: Academic Press). Brain tissues were then frozen by immersion in 2-methylbutane surrounded by dry ice, and stored at -80°C.

RNA quality control: Quality control of the RNA was performed using the RNA 6000 Nano chip (Agilent) on an Agilent Bioalyzer 2100 obtaining RIN values between 8.7 - 9.8. RNA libraries: Libraries were prepared from 500 ng of total RNA using the TruSeq stranded mRNA library preparation kit (Illumina, #20020594) with TruSeq RNA Single Indexes (Illumina, #20020492 and #20020493) according to the manufacturer's instruction reducing the RNA fragmentation time to 4.5 minutes. Prepared libraries were analysed on a DNA 1000 chip on the Bioanalyzer and quantified using the KAPA Library Quantification Kit (Roche, #07960204001) on an ABI 7900HT qPCR instrument (Applied Biosystems). Sequencing was performed with 2x50 bp paired-end reads on a HiSeq 2500 (Illumina) using HiSeq v4 sequencing chemistry.
*Bioinformatic* analysis: Raw sequencing reads in the fastq files were mapped with STAR version 2.5.3a (Dobin, A. et al. 2013. STAR: Ultrafast universal RNA-seq aligner. Bioinformatics 29, 15-21) to the Gencode release 17 based on the GRCm38.p6 reference genome and the corresponding GTF file. The table of counts was obtained with FeatureCounts function in the package subread, version 1.5.1.(Liao, Y. et al. 2014. FeatureCounts: An efficient general purpose program for assigning sequence reads to genomic features. Bioinformatics 30, 923-930). The differential expression gene analysis (DEG) was assessed with voom+limma in the limma package version 3.30.13 (Smyth, G.K. 2005. limma: Linear Models for Microarray Data. In Bioinformatics and Computational Biology Solutions Using R and Bioconductor, (New York: Springer-Verlag), pp. 397-420) and R version 3.4.3. Genes having less than 10 counts in at least 5 samples were excluded from the analysis. Raw library size differences between samples were treated with the weighted "trimmed mean method" TMM (Robinson, M.D., and Oshlack, A. 2010. A scaling normalization method for differential expression analysis of RNA-seq data. Genome Biol. 11, R25) implemented in the edgeR package (Robinson, M.D. et al. 2009. edgeR: A Bioconductor package for differential expression analysis of digital gene expression data. Bioinformatics 26, 139-140). The normalized counts were used in order to make unsupervised analysis, PCA and clusters. For the differential expression (DE) analysis, read counts were converted to log2-counts-per-million (logCPM) and the mean-variance relationship was modelled with precision weights using voom approach in limma package.

### Example 8: Statistical analyses

Firstly, normal distribution and homogeneity of variances were tested. Results are expressed as number and frequencies for categorical variables, mean and standard deviation (SD) for normal distributed continuous variables and median and interquartile range [IQ] for non-normal distributed continuous variables. To determine differences between study groups, X² for categorical variables were used, unpaired Student's t-test in normal quantitative and Mann-Whitney U test for non-normal quantitative variables. Spearman or Pearson analysis was used to determine the correlation between quantitative variables. Theses statistical analyses were performed with SPSS, version 19 (SPSS, Inc, Chicago, IL).
Differential abundance analyses for taxa and functions associated to the memory tests and brain areas volumes were performed using the DESeq2 R packages, adjusting for age, body mass index, sex, education years, and Patient Health Questionnaire (PHQ)-9 scores. Fold change associated with a unit change in the corresponding test and adjusted p-values are plotted for each taxon. Significantly different taxa are coloured according to phylum. OTUs and bacterial functions were previously filtered so that only those with more than 10 reads in at least two samples were selected.
Metabolomics data were also analysed using machine learning (ML) methods. Omics datasets are usually composed of high-dimensional data with many redundant, non-informative and noisy features, *i.e.,* not related to the outcome, with complex correlation patterns. Therefore, feature selection, plays a crucial role in omics data analysis. In this context, ML methods, such as random forest (RF), are promising computational approaches for feature selection in high-dimensional omics data sets. ML tree-based algorithms are particularly well-suited to this aim. Thus, variable selection tree-based methods have shown to perform better than classic regression-based methods in large datasets (Sanchez-Pinto, L.N. et al. 2018. Comparison of variable selection methods for clinical predictive modelling. Int. J. Med. Inform. 116, 10-17). When the main goal is building a predictive model, variable selection techniques designed to identify a minimal set of strongest predictors associated with the outcome are used **(minimal-optimal problem).** However, if the objective involves providing a more holistic pictures of the underlying mechanisms, networks and pathways involved in pathophysiological or metabolic processes, **all-relevant variable selection methods,** which include weak, correlated and redundant features, but avoid inclusion of uninformative variables, are preferred (Shi, L. et al. 2019. Variable selection and validation in multivariate modelling. Bioinformatics 35, 972-980). Therefore, an all-relevant machine learning variable selection strategy was adopted applying two random forest-based methods, the Boruta algorithm (Kursa, M.B., and Rudnicki, W.R. 2010. Feature selection with the boruta package. J. Stat. Softw. 36, 1-13) and the Variable Importance Testing Approach (VITA) method (Janitza, S., Celik, E., and Boulesteix, A.L. 2018. A computationally fast variable importance test for random forests for high-dimensional data. Adv. Data Anal. Classif. 12, 885-915). The Boruta and Vita approaches have been recently proposed as the two best-performing variable selection methods making use of RF for high-dimensional omics datasets. RF is an ensemble machine learning method based on "growing" many classification or regression trees. The advantage of the RF is that the observations not used for the construction of a specific tree (termed *out-of-bag* (OOB) observations) may be used to estimate the variable importance measure (VIM). Among the several VIMs, the permutation variable importance has shown to be the most reliable. However, a drawback of VIMs in RF is that they are not directly related to the statistical significance and there is no statistical test that discriminates between relevant and non-relevant features. Boruta and Vita are two RF-based approaches that deal with this issue. The **Boruta algorithm** is a wrapper algorithm that performs feature selection based on the learning performance of the model. The main idea behind this approach consists in:
**a) Randomization.** Create a duplicate copy of the original features randomly permutate across the observations (the so-called shadow features) to remove their correlation with the response;
**b) Model building.** Add the shadow feature to the original predictor feature data set, built a RF with the extended data set, and compute the normalized permutation importance (*Z*) scores for each predictor and shadow feature;
**c) Statistical testing.** Find the maximum normalized importance among the shadow attributes (MZSA) and compare it with each original predictor feature using a Bonferroni corrected two-tailed binomial test. Predictor features with significantly higher, significantly lower, or non-significantly different *Z* scores than expected at random compared to the MZSA are deemed important, unimportant, or tentative, respectively.
**d) Iteration.** Unimportant and shadow features are removed and the previous steps are repeated until the status of all features is decided or a predefined number of iterations has been performed. The Boruta algorithm with 500 iterations was run, a confidence level cut-off of 0.005 for the Bonferroni adjusted *p*-values, 5000 trees to grow the forest (*ntree*), and a number of features randomly sampled at each split given by the rounded down number of features/3 (the *mtry* recommended for regression).

The **Vita algorithm** is based on the assumption that most variables in omics data sets are non-relevant for the biological question and can be used to approximate the unknown null distribution of variable importance scores to be able to select relevant variables based on p-values (Janitza, S., Celik, E., and Boulesteix, A.L. 2018. A computationally fast variable importance test for random forests for high-dimensional data. Adv. Data Anal. Classif. 12, 885-915. First, the VIM for all features are obtained. The importance measure in the vita algorithm is not based on the "standard" permutation variable importance calculated using the OOB samples, but uses a strategy inspired in the cross-validation (CV) procedure, which is not based on the OOB observations, to obtain the CV permutation variable importance (CVPVI). The method randomly splits the data in a total of *k*-folds of equal size. For each *i*-fold, a RF is trained using all samples that are not part of the *i*-test set, and the response variable is predicted for the samples in the *i-*test set. The procedure is repeated after permutating n times the values of the predictor variables. The permutation variable importance is calculated as the average difference in the prediction errors between the original data and the permutations, and the CVPVI is the average over all *k*-fold-specific permutation variable importance. Second, taking into account that for non-relevant features the change in accuracy is only due to random variations and thus it does not change (zero CVPVI) or slightly increases (negative CVPVI) when not using the variable for prediction, the non-positive CVPVI values are used compute the a symmetric null distribution of CVPVI scores around zero for non-relevant features by mirroring them on the y-axis. From this approximated null distribution, p-values can be calculated. As the null distribution is obtained from non-relevant features, this testing approach is only suitable for data sets with a large number of variables without effect. A 7-fold CV was used in the calculations, and 10 permutations. P-values were then corrected using the Benjamini-Hochberg procedure for FDR.

## Claims

1. A gut microbiota composition, comprising at least one species of genus *Clostridium* (family Clostridiaceae), *Lactobacillus* (family Lactobacillaceae), *Megamonas* and *Anaerovibrio* (family Selenomonadaceae), or *Roseburia* (family Lachnospiraceae) of phylum Firmicutes,
for use in the prevention and/or treatment of a mental disorder with memory impairment in a subject.

2. The gut microbiota composition according to claim 1, comprising a combination of at least one species of each genus.

3. The gut microbiota composition according to claims 1 or 2, in which said species are *Clostridium sp. 27_14, Clostridium sp. CAG:230, Clostridium sp. CAG440, Lactobacillus rhamnosus, Megamonas funiformis, Anaerovibrio sp. JC8* and *Roseburia sp. CAG197.*

4. A gut microbiota composition, comprising at least one species of genus *Clostridium* (family Clostridiaceae); *Ruminococcus* (family Ruminococcaceae); *Megamonas* or *Anaerovibrio* (family Selenomonadaceae); *Acetitomaculum* (family Lachnospiraceae); or the species *Schwartzia succinivorans* (family Selenomonadaceae) or *Firmicutes bacterium CAG:95,*
for use in the prevention and/or treatment of a mental disorder with memory impairment in a subject, in which said memory impairment is an impairment in verbal memory.

5. The gut microbiota composition according to claim 4, comprising a combination of at least one species of each genus *Clostridium; Ruminococcus; Megamonas, Anaerovibrio, Acetitomaculum,* and of *Schwartzia succinivorans* and *Firmicutes bacterium CAG:95.*

6. The gut microbiota composition according to claims 4 or 5, in which said species are *Clostridium sp.44_14, Ruminococcus sp. CAG:379, Megamonas hypermegale, Anaerovibrio lipolyticus, Acetitomaculum ruminis, Schwartzia succinivorans* and *Firmicutes bacterium CAG:95.*

7. A gut microbiota composition, comprising at least one species of genus *Ruminococcus* (family Ruminococcaceae), *Megasphaera* (family Veillonellaceae) or *Anaerovibrio* (family Selenomonadaceae),
for use in the prevention and/or treatment of a mental disorder with memory impairment in a subject, in which said memory impairment is an impairment in immediate memory.

8. The gut microbiota composition according to claim 7, comprising a combination of at least one species of each genus.

9. A gut microbiota composition, comprising at least one species of *Lactobacillus rhamnosus* or *L. helveticus,*
for use in the prevention and/or treatment of a mental disorder with memory impairment in a subject, said memory impairment is an impairment in non-spatial memory.

10. The gut microbiota composition according to claim 9, comprising a combination of the species of *Lactobacillus rhamnosus* and *L. helveticus.*

11. A gut microbiota composition, comprising at least one species of genus *Tissierella* (family Clostridiaceae,
for use in the prevention and/or treatment of a mental disorder with memory impairment in a subject, in which said memory impairment is an impairment in the memory function.

12. A gut microbiota composition, comprising at least one species of *Dakarella massiliensis* or *Sutterella* (family Sutterellaceae), or *Leuconostoc* (family Leuconostocaceae).
for use in the prevention and/or treatment of a mental disorder with memory impairment in a subject, in which said memory impairment is an impairment in the memory function.

13. The gut microbiota composition according to claim 12, comprising a combination of the species of *Dakarella massiliensis,* and genera *Sutterella* and *Leuconostoc.*

14. The gut microbiota composition according to claim 12 or 13, in which said species are *Dakarella massiliensis, Sutterella sp. CAG:351,* and *Leuconostoc_uc.*

15. The gut microbiota composition according to any one of the preceding claims, in which said microbiota composition further comprises at least one species of the Sporolactobacilliaceae, Anaerolineae, Acidobacteria, Blastocatellia, Odoribacteraceae, Ruminococcaceae, Lachnospiraceae families, or combinations thereof.

16. The gut microbiota composition of any one of the preceding claims, comprising an intestinal fungus of the phylum Microsporidia.

17. A gut microbiota composition according to any one of claims 1 to 16, in which said mental disorder with memory impairment is Parkinson's disease or Alzheimer's disease.

18. A gut microbiota composition according to any one of claims 1 to 16, in which said mental disorder with memory impairment is dementia.

19. A gut microbiota composition according to any one of the preceding claims, in which said subject is a human.

20. The gut microbiota composition according to any one of the preceding claims, in which said composition comprises at least 100,000 microorganisms per mL.

21. The gut microbiota composition according to claim 20, in which said composition comprises at least 500,000 microorganisms per mL.

22. The gut microbiota composition according to any one of the preceding claims, comprising at least a pharmaceutically acceptable excipient.

23. The gut microbiota composition according to any one of the preceding claims, in which said composition is presented in a dosage form able to be administered orally.

24. The gut microbiota composition according to claim 23, in which said composition is presented in liquid form.

25. The gut microbiota composition according to claim 23 or 24, in which said composition is presented in the form of a pill, tablet, a capsule, solution, suspension, syrup, or a food containing the probiotic bacteria.
